# EUROPEAN PATENT SPECIFICATION

(11) **EP 3 641 534 B1**
(45) Date of publication and mention of the grant of the patent: **20.10.2021**
(21) Application number: 18738018.3
(22) Date of filing: 18.06.2018
(51) Int. Cl.: C12Q 1/58, G01N 31/22, G01N 33/62, A01J 5/013, G01N 33/04

(54) **ARRANGEMENT AND METHOD FOR UREA DETERMINATION**
ANORDNUNG UND VERFAHREN ZUR HARNSTOFFBESTIMMUNG
AGENCEMENT ET PROCÉDÉ DE MESURE POUR LA DÉTERMINATION D'URÉE

(30) Priority: 20.06.2017 SE 1750791
(43) Date of publication of application: 29.04.2020
(73) Proprietor: DeLaval Holding AB, 147 21 Tumba (SE)
(72) Inventor: DALLERUP RASMUSSEN, Claus, 147 21 Tumba (SE); TRIER HALD, Jonas, 147 21 Tumba (SE)
(74) Representative: Lilliehorn, Tobias
(86) International application number: PCT/SE2018/050640
(87) International publication number: WO 2018/236272

(56) References cited:
- EP-A1- 0 805 350
- EP-A2- 0 896 222
- FR-A1- 2 731 276
- NORMAN F. SHEPPARD ET AL: "Model of an immobilized enzyme conductimetric urea biosensor", BIOSENSORS & BIOELECTRONICS, vol. 11, no. 10, 1 January 1996 (1996-01-01), pages 967-979, XP055506103,
- CULLEN D C ET AL: "Multi-analyte miniature conductance biosensor", ANALYTICA CHIMICA ACTA, ELSEVIER, AMSTERDAM, NL, vol. 231, 1 January 1990 (1990-01-01), pages 33-40, XP026725067, ISSN: 0003-2670, DOI: 10.1016/S0003-2670(00)86394-1 [retrieved on 1990-01-01]

## Description

### TECHNICAL FIELD

This document discloses an arrangement and a method. More particularly, an arrangement and a method are described, for measuring urea in a milk sample.

### BACKGROUND

Urea is a nitrogen compound that may be present in the milk of an animal. The amount of urea in the milk is related to the amount of urea circulating in the blood of the animal, which in turn is affected by the amount of protein eaten by the animal. By measuring the amount of urea in the milk, an approximate indicator of the dietary protein supply to the animal is obtained.

High levels of urea in the milk (i.e. a urea level exceeding a threshold limit such as e.g. >12-14 mg/ dl) may indicate excess dietary protein or an imbalance of ruminal protein, while a low urea level (e.g. < 8-10 mg/ dl) may indicate a possible dietary protein deficiency. The threshold limits may be different for different breeds, body weight and other parameters of the animal. Further, high levels of urea in the milk, exceeding a threshold limit, may affect cheese production.

By monitoring the urea level in milk of diary animals, the dietary protein supply of the animal may be adjusted in order to optimise milk yield of the animal, while avoiding excess protein feeding. Such monitoring is advantageous to do continuously, or at regular intervals, at the farm, in association with milking of the animal.

However, the legacy methods available for measuring urea level are mainly designed for laboratory usage, making it problematic for the average farmer to use during daily work at the farm, as the work typically is intense and the farmer may have to prioritise other tasks.

FR2731276 A1 discloses a strip impregnated with reagents suitable to react quantitatively and proportionally with the urea present in milk for estimating the level of urea in milk.

It would for these reasons be advantageous for the farmer, if the urea monitoring of milk samples of different animals could be automated, and thereby minimising or at least reducing the manual work effort of the farmer.

It would be desired to find a way to assist the farmer in analysing his/ her animals and enhance production at the farm.

### SUMMARY

It is therefore an object of this invention to solve at least some of the above problems and facilitate for an operator/ farmer to detect presence and/ or measure amount of urea in a milk sample of an animal.

According to a first aspect of the invention, this objective is achieved by an arrangement according to claim 1 configured to measure urea in a milk sample of an animal. The arrangement comprises a reference indicator, impregnated with a halochromic substance configured to adjust colour of the reference indicator, based on level of hydroxide ions of a first sub sample of the milk sample, when added to the reference indicator. Furthermore, the arrangement also comprises a measurement indicator, impregnated with the halochromic substance of the reference indicator, and prepared with an enzyme configured to convert urea of a second sub sample of the milk sample, when added to the measurement indicator, into at least one chemical substance, which in turn will influence the level of hydroxide ions of the second sub sample. In addition, the arrangement further comprises a sensor configured to capture a first image of the reference indicator, and a second image of the measurement indicator. The arrangement further also comprises a control unit configured to determine a colour difference between the first image of the reference indicator and the second image of the measurement indicator; and configured to convert the determined colour difference into an amount of urea in the milk sample of the animal.

According to a second aspect of the invention, this objective is achieved by a method according to claim 8 to measure urea in a milk sample of an animal. The method comprises extracting the milk sample from the animal. Further, the method also comprises adding a first sub sample of the extracted milk sample to a reference indicator, impregnated with a halochromic substance configured to adjust colour of the reference indicator, based on level of hydroxide ions of the first sub sample of the milk sample. The method in addition also comprises adding a second sub sample of the milk sample to a measurement indicator, impregnated with the halochromic substance of the reference indicator, and prepared with an enzyme configured to convert urea of the second sub sample, into at least one chemical substance, which in turn will influence the level of hydroxide ions of the second sub sample. Also, the method further comprises capturing a first image of the reference indicator. The method furthermore comprises capturing a second image of the measurement indicator. In addition, the method comprises comparing the colour of the captured first image with the colour of the captured second image. Further, the method additionally also comprises detecting a colour difference between the captured first image and the captured second image. The method comprises converting the detected colour difference into an amount of urea in the milk sample of the animal.

Thanks to the described aspects, by providing one sub sample each, of the milk sample, to the reference indicator and the measurement indicator respectively, a reference of the pH level at the current conditions is obtained. The problems associated with knowing pH level of the animal at the moment of the test, which typically vary based on a plurality of factors between individual animals, are thereby omitted, and it becomes possible to determine the urea content of the milk sample with a degree of accuracy which is considerably more accurate than by legacy methods. Thus, an easy and economic method of determining urea in milk is obtained, enabling frequent urea analysis on herd animals to improve farm management.

Other advantages and additional novel features will become apparent from the subsequent detailed description.

### FIGURES

Embodiments of the invention will now be described in further detail with reference to the accompanying figures, in which:
- **Figure 1**: illustrates an example of an arrangement for measuring urea in a milk sample of an animal.
- **Figure 2**: illustrates an example of an arrangement for measuring urea in a milk sample of an animal.
- **Figure 3A**: illustrates a cassette, according to an embodiment.
- **Figure 3B**: illustrates a second cassette, according to an embodiment.
- **Figure 4**: illustrates a method according to an embodiment.
- **Figure 5**: is an illustration depicting an arrangement according to an embodiment.

### DETAILED DESCRIPTION

Embodiments of the invention described herein are defined as an arrangement and a method, which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

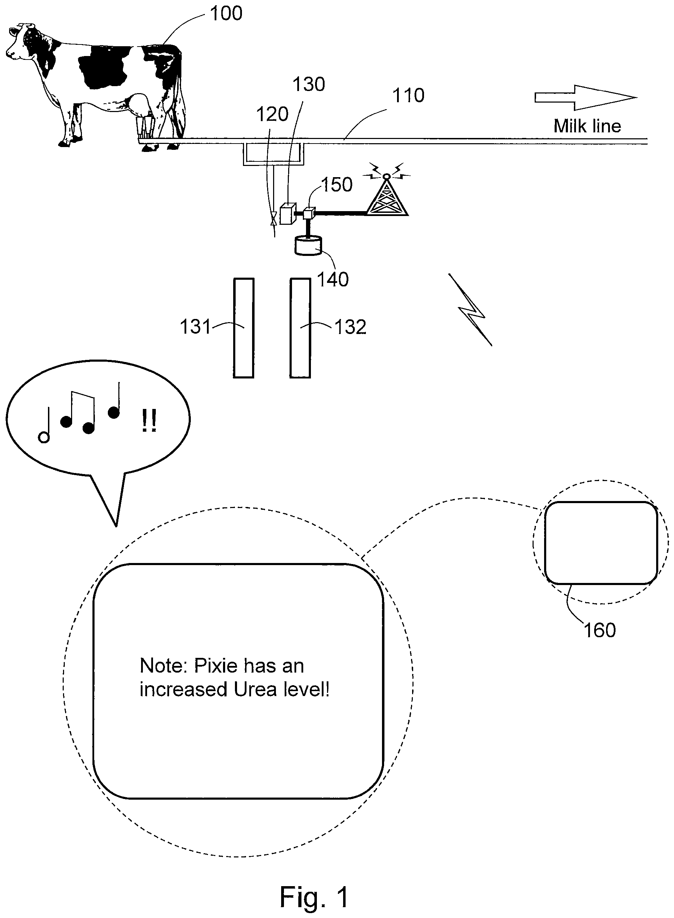

**Figure 1** illustrates a scenario with an animal **100** which may be comprised in a herd of dairy animals at a dairy farm.

"Animal" may be any arbitrary type of domesticated female milk producing and/ or meat producing mammal such as cow, goat, sheep, horse, camel, dromedary, primate, dairy buffalo, donkey, reindeer, llama, yak, elk etc.

Milk of the animal 100 may be extracted by a milking equipment **110** such as e.g. a milking robot or other milking arrangement. A representative milk sample may be prepared. The prepared milk sample may then, via an applicator **120,** be applied onto a reference indicator **131** and a measurement indicator **132,** respectively.

The term "milk sample" designates samples of milk and related products, such as raw milk, whole milk, skim milk, cream, re dissolved and/ or resuspended powdered milk. This sample may or may not be homogenised in different embodiments. In accordance with an embodiment, the sample may be a sample of raw milk, extracted directly from a milking equipment 110.

The reference indicator 131 and/ or the measurement indicator 132 respectively may comprise, or be based on a paper stick such as a pH test strip or a universal indicator paper. The reference indicator 131 and/ or the measurement indicator 132 may be impregnated with, such that an added milk sample is evenly absorbed by the reference indicator 131 and/ or the measurement indicator 132, and/ or being prepared with a non-bleed feature.

The reference indicator 131 is impregnated with a halochromic substance configured to adjust colour of the reference indicator 131, based on level of hydroxide ions of a first sub sample of the milk sample, when added to the reference indicator 131. Thereby, the colour nuance of the reference indicator 131 may indicate the potential of Hydrogen (pH) of the milk sample.

The halochromic substance comprises a material which changes colour when a pH change occurs. Thus, the halochromic substance becomes a pH indicator. The halochromic substance may detect alterations in urea and thereby the acidity of the milk sample. The colour obtained may be compared with the colour obtained when the reference indicator 131 is exposed to a solution with known pH. The pH of the milk sample may then be estimated.

The colour change of halochromic substances in the reference indicator 131 occur when the chemical binds to existing hydrogen and hydroxide ions in solution. Such bonds result in changes in the conjugated systems of the molecule, or the range of electron flow. This alters the amount of light absorbed, which in turn results in a visible change of colour. Halochromic substances do not display a full range of colour for a full range of pH because, after certain acidities, the conjugated system will not change. The various shades result from different concentrations of halochromic molecules with different conjugated systems.

A sensor **130** may capture an image of the reference indicator 131, after a predetermined or configurable incubation time, such as e.g. 5 minutes, 10 minutes, etc. The sensor 130 may comprise a camera, a video camera or similar device configured to capture an image. The sensor 130 may comprise e.g. a Charge-Coupled Device (CCD), an Active-Pixel Sensor (APS), a Complementary Metal-Oxide-Semiconductor (CMOS) sensor or other similar arrangement in different embodiments.

The length of the incubation time may be influenced by the temperature; i.e. the higher temperature, the shorter will the chemical reaction time become (within reasonable limits). The temperature may be kept at the same level for both the reference indicator 131 and the measurement indicator 132 during the incubation time. Thereby, by keeping the temperature the same both for the reference indicator 131 and the measurement indicator 132, the difference between the colour change of the respective indicators 131, 132 may remain the same, independently of the temperature. However, in some embodiments, the temperature of the environment surrounding the reference indicator 131 and the measurement indicator 132 may be kept at a constant level such as e.g. 25 degrees; or within an interval such as e.g. 22 degrees and 28 degrees (arbitrary, non-limiting examples) in some embodiments; at least during the incubation time period.

The sensor 130 may also capture an image of the measurement indicator 132 after the predetermined or configurable incubation time which may be the same as for the reference indicator 131, or different, in different embodiments. The measurement indicator 132 is impregnated with the halochromic substance of the reference indicator 131 described above, and prepared with an enzyme, e.g. a hydrolytic enzyme such as Urease or Allophanate hydrolase, configured to convert urea of a second sub sample of the milk sample, when added to the measurement indicator 132, into at least one chemical substance, which in turn will influence the level of hydroxide ions of the second sub sample. In some embodiments, the buffer level of the enzyme, such as the hydrolytic enzyme, Urea, may in some embodiments be lower than a predetermined or configurable threshold level. Thereby, the previously described effect of converting urea to the chemical substance which in turn influences the level of hydroxide ions of the milk sample becomes enhanced.

A hydrolytic enzyme, sometimes also referred to as hydrolase, is an enzyme that catalyses the hydrolysis of a chemical bond. Urease is a hydrolytic enzyme that catalyses the hydrolysis of urea in the milk sample into carbon dioxide and ammonia.

Thus, the enzyme of the measurement indicator 132 may catalyse the hydrolysis of urea (if any) in the milk sample, to produce ammonia and carbamate. The carbamate produced may subsequently degrade by spontaneous hydrolysis to produce another ammonia and carbonic acid, which increases the pH level of the milk sample. Thereby, in case urea is present in the second sub sample of the milk sample applied to the measurement indicator 132, the colour of the measurement indicator 132 will change to another colour nuance than the reference indicator 131, which serves as a reference.

A problem when measuring pH levels of milk samples is that the variation for various reasons is very large between different individual animals, but also between different milk samples of the same animal, extracted at different moments in time. Generally, the pH (potential of Hydrogen) levels of milk samples may vary between e.g. 6.1-6.7 pH. By using a temporary reference indicator 131 of the pH level for each urea test by the measurement indicator 132, the problem of variating pH levels is omitted, as a reference is created for each measurement.

The captured images of the reference indicators 131, 132 may be forwarded by the sensor 130 to a control unit **150.** The control unit 150 is configured to determine a colour difference between the first image of the reference indicator 131 and the second image of the measurement indicator 132. The control unit 150 may thus compare the colour nuance of the captured image of the reference indicator 131 with the colour nuance of the captured image of the measurement indicator 132. In case there is a colour nuance distinction between the respective captured images, the reason is that urea in the milk sample has been converted into ammonia and carbonic acid, which increases the pH level and thereby creates the colour difference between the samples. The control unit 150 is thus configured to convert the determined colour difference into an amount of urea in the milk sample of the animal 100.

Further, the control unit 150 may detect the colour difference between the captured first image and the captured second image by converting the detected colour/ colour nuance of the measurement indicator 132 into a pH level by making a comparison with a reference chart where colours/ colour nuances are mapped with different pH levels. Also, the corresponding procedure may be performed by the control unit 150 for the reference indicator 131. Thus, the detected colour/ colour nuance of the captured first image of the reference indicator 131 may be converted into a pH level by making a comparison with the reference chart. The estimated pH level of the reference indicator 131 may then be subtracted from the estimated pH level of the measurement indicator 132. The estimated pH level difference is a result from the conversion by the enzyme on the measurement indicator 132, of the urea in the second sub sample of the milk sample. Thereby, the detected colour difference between the reference indicator 131 and the measurement indicator 132 may be converted into an amount of urea in the milk sample of the animal 100 by converting the estimated pH difference into an amount of urea. The conversion may be made e.g. by a check in a look-up table where a pH difference is mapped with a corresponding amount of urea.

The control unit 150 may further store the result of the urea measurement in a database **140,** associated with e.g. an identity of the animal 100 and a time reference. The database 140 may also store other measurements and/ or data related to the animal 100, such as milk yield, e.g. measured by the milk flow meter, activity, breed, parity, rumination, lactation, resting, feed intake, energy balance, Days In Milk, milk production, age and possibly other similar animal status related parameters.

Further, in some embodiments, the control unit 150 may transmit information to a communication device **160** of an operator via a wired or wireless communication interface.

Such wireless communication interface may comprise, or at least be inspired by wireless communication technology such as Wi-Fi, Wireless Local Area Network (WLAN), Ultra Mobile Broadband (UMB), Bluetooth (BT) to name but a few possible examples of wireless communications in some embodiments. The communication may alternatively be made over a wireless interface comprising, or at least being inspired by radio access technologies such as e.g. 3GPP LTE, LTE-Advanced, E-UTRAN, UMTS, GSM, GSM/ EDGE, WCDMA, Time Division Multiple Access (TDMA) networks, Frequency Division Multiple Access (FDMA) networks, Orthogonal FDMA (OFDMA) networks, Single-Carrier FDMA (SC-FDMA) networks, Worldwide Interoperability for Microwave Access (WiMax), or Ultra Mobile Broadband (UMB), High Speed Packet Access (HSPA) Evolved Universal Terrestrial Radio Access (E-UTRA), Universal Terrestrial Radio Access (UTRA), GSM EDGE Radio Access Network (GERAN), 3GPP2 CDMA technologies, e.g., CDMA2000 1x RTT and High Rate Packet Data (HRPD), or similar, just to mention some few options, via a wireless communication network.

The communication device 160 of the farmer may be e.g. a cellular mobile telephone, a stationary or portable computing device, a computer tablet, a display, a pair of intelligent glasses, a smart contact lens, an augmented reality device, a smart watch or similar device having a user interface and wireless communication ability.

The information communicated with the operator may comprise e.g. visual information, an audio message, a tactile signal or a combination thereof, encouraging the operator to further investigate the reasons for the detected deviation in result. In case a plurality of people is working with the herd, a broadcast may be made to the plurality of operators and their respective associated communication device 160, in some embodiments.

The operator may be e.g. a farmer or other person working at a farm; or a veterinarian, agronomist, dietician, biologist, zoologist, ecologist, mammologist, domestic animal researcher, zookeeper or other similar human, temporarily, accidently or permanently visiting the farm. The "farm" as the term herein is used may be a barn, a ranch, a stable or other similar agricultural structure for keeping animals 100.

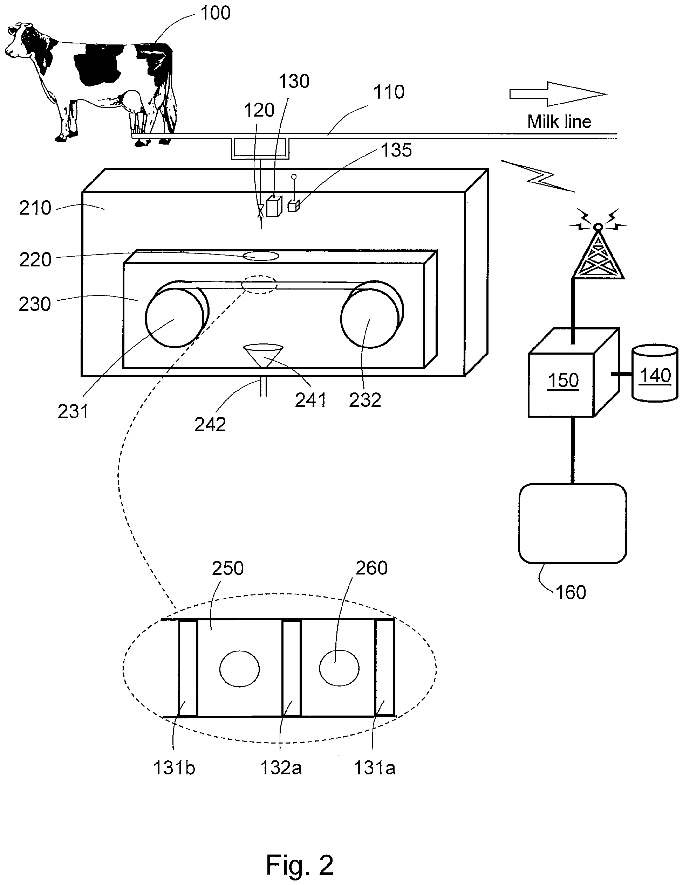

**Figure 2** illustrates yet an example of an arrangement for measuring urea in a milk sample of an animal 100. A milk sample may be extracted from a milking equipment 110, such as e.g. a milking robot, a manual bucket milker, a (rotary) milking parlour, etc.

In some embodiments, various equipment of the arrangement previously described in Figure 1, such as e.g. an applicator 120, a sensor 130, one or several pumps, a tube element for attachment to the interface to the milking equipment 110, a motor, a communication device 135, etc., may be comprised in a service module **210**.

The service module 210 may be releasably inserted into the milking equipment 110 in some embodiments. Thus, there may be an interface between the milking equipment 110 and the service module 120 for providing milk and possibly electricity via the milking equipment 110 to the service module 210.

A cassette **230** may be detachably inserted into the service module 210. The cassette 230 may comprise a carrier tape **250** with at least one reference indicator 131 and/ or at least one measurement indicator 132.

The carrier tape 250 may be arranged on a first spool **231** and a second spool **232,** which spools 231, 232 may be arranged to cooperate with a cassette external motor for positional adjustment of the carrier tape 250. The cassette external motor may be comprised in the service module 210 in some embodiments. The sensor 130 of the service module 210 may capture an image of the reference indicator 131 and/ or the measurement indicator 132 through an inspection window **220.** Based on this image or images, the cassette external motor may adjust the carrier tape 250 for positioning the reference indicator 131 and/ or the measurement indicator 132 on the carrier tape 250, on which a new test is to be made, in relation to an applicator 120 of the cassette 230.

Thereby, a milk sample of the animal 100 may be extracted from the animal 100 by the milking equipment 110 and provided via the service module 210 to the reference indicator 131 and/ or the measurement indicator 132. The reference indicator 131 and/ or the measurement indicator 132 may change colour and/ or colour nuance when exposed to the milk sample, as previously described. The captured image of the reference indicator 131 and/ or the measurement indicator 132 may then be analysed by a control unit 130. The presence and/ or quantity of urea in the milk sample may thereby be determined.

The service module 210 may comprise a control unit 150 and/ or a database 140 in some embodiments. However, in other embodiments as illustrated in Figure 2, the control unit 150 and/ or the database 140 may be external to the service module 210.

The cassette 230 may also comprise a liquid insertion connection configured to receive the milk sample of the animal 100 from the applicator 120 of the service module 210.

The carrier tape 250 of the cassette 230 may in some embodiments comprise a flush hole **260.** To distinguish the milk samples of the different animals 100, it may be avoided that milk from a previously milked animal 100 remains in the tubing of the service module 210, and/ or cassette 230 by flushing the respective tubing with milk of the animal 100, from which a new sample is to be taken. The carrier tape 250 may then be forwarded so that milk from the animal 100 could be flushed via the flush hole 260, before the carrier tape 250 again is forwarded for putting the reference indicator 131 and/ or the measurement indicator 132 in position for receiving a milk sample from the subsequently following animal.

The milk provided to the cassette 230, either for the milk sample applied on the reference indicator 131 and/ or the measurement indicator 132, or the milk flushed through the flush hole 260, may be evacuated from the cassette 230 via an aperture **241** of the cassette 230, and a drainage **242** of the service module 210. The evacuated milk may in some embodiments be returned back to the milk line; in other embodiments, the evacuated milk may be dissipated to a sewer or otherwise further evacuated from the farm.

In some embodiments, the cassette 230 may be sealed from the environment and thereby create a climate chamber, wherein a climate environment prevails in the cassette 230. The cassette 230 thereby becomes isolated from environmental impact of dust, dirt, liquids, etc., of the farm. Further, temperature of the reference indicator 131 and/ or the measurement indicator 132 may be kept at a constant level such as e.g. 25 degrees; or within an interval such as e.g. 22 degrees and 28 degrees (arbitrary, non-limiting examples).

The cassette 230, in some embodiments, may comprise a sealing tape configured to seal the reference indicator 131 and/ or the measurement indicator 132 on the carrier tape 250. The reason is that it is important that milk from a first animal 100 does not soak the reference indicator 131 and/ or the measurement indicator 132 on which a subsequent animal is to use for urea test, as the milk from the first animal may contaminate the reference indicator 131 and/ or the measurement indicator 132. For this reason, the cassette 130 may further comprise a de-sealer, configured to remove the sealing tape from the reference indicator 131/ measurement indicator 132 while being adjusted into a position aligned with the applicator 120.

Returning to the service module 210; the service module 210 may comprises at least one motor configured to be engaged with at least one of a first spool 231 or a second spool 232 of the cassette 230, in order to adjust the position of the reference indicator 131 and/ or the measurement indicator 132 on the carrier tape 250, in relation to the inspection window 220. The motor may propel a first rotary driving means and/ or a second rotary driving means; which first rotary driving means and/ or second rotary driving means may interact with the first spool 231 and/ or the second spool 232 of the cassette 230, whereon the carrier tape 250 is arranged. The motor may then adjust the position of the reference indicator 131 and/ or the measurement indicator 132 on the carrier tape 250, in relation to the inspection window 220 of the cassette 230 by rotating the first spool 231 and/ or the second spool 232 of the cassette 230 with the first rotary driving means and/ or second rotary driving means.

The motor may comprise an electric motor, such as e.g. an Alternating Current (AC) motor in some embodiments. The currency may be supported from an external source of energy. In some embodiments, the motor may be a Direct Currency (DC) motor.

Further, the service module 210 also comprises a tube element configured to receive the milk sample of the animal 100 via the milking equipment 110 and in some embodiments, provide the milk sample to a needle of the cassette 230 via a liquid insertion connection of the cassette 230. Thereby, there may be an interface between the service module 210 and the cassette 230, wherein the tube element may be connected to the liquid insertion connection of the cassette 230.

The service module 210 may in addition comprise at least one pump configured to act on the tube element for providing the milk sample to applicator 120; or alternatively the needle of the cassette 230. The pump may thus act on the tube element to get the milk sample to propagate through the tube element, via the liquid insertion connection of the cassette 230 to reach the needle of the cassette 230, in some embodiments.

Further, the service module 210 may comprise a drainage 242 arranged to receive liquid evacuated from the cassette 230 via an aperture 241 of the cassette 230, and to evacuate the liquid from the service module 210.

An advantage of the disclosed solution, by making a division between a service module 210 comprising sensor 130, motor, pumps and other electronics and/ or apparatuses; and a cassette 230 comprising disposable material, the solution becomes very easy to use for the operator.

The cassette 230 may comprise the reference indicator 131 and/ or the measurement indicator 132 for supporting the farm for a certain predetermined period of time. Before the end of that time period, a supplier may provide a new cassette 230 to the farm/ operator, which the operator easy may put into the service module 210, without having to interact with the sensible electronics of the service module 210. The used cassette 230 may then be disposed.

In case a hardware failure or other malfunction occur, the operator may remove the service module 210 from the milking equipment 110 (and also remove the cassette 230 from the service module 210) and provide the service module 210 to a service supplier for reparation/ adjustment. During the time period the service module 210 is on repair, the operator may borrow another service module 210 from the service supplier. Thereby, urea values of the animals 100 may be monitored without interruptions, also when the service module 210 or any part thereof is malfunctioning. Also, as no external technician is required to visit the farm, neither for changing the disposable cassette 230, nor for analysing errors in the service module 210, costs for service and maintenance are minimised or at least reduced.

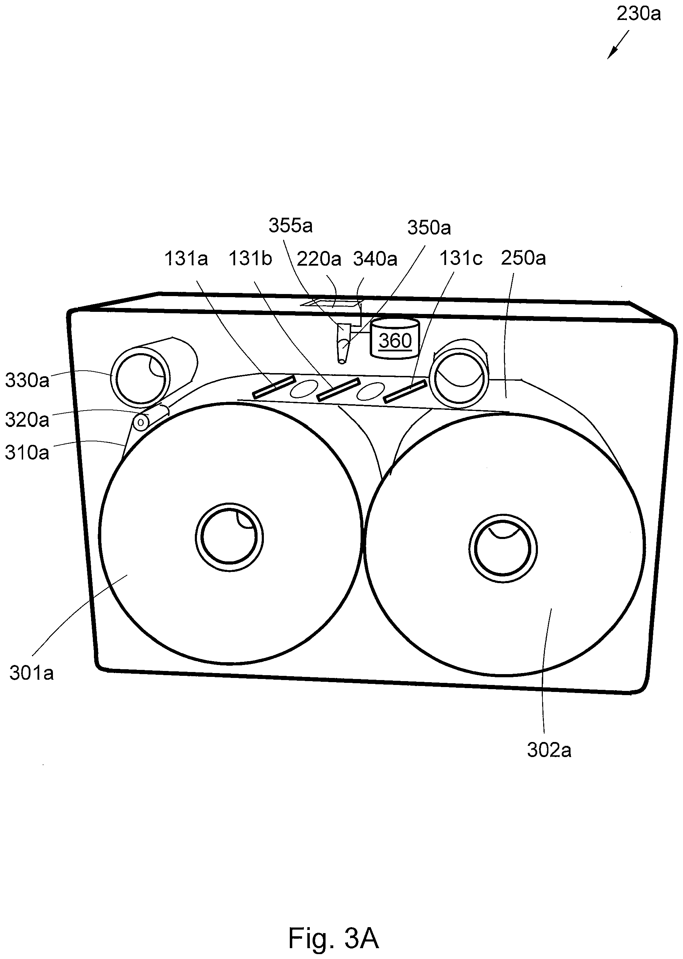

**Figure 3A** illustrates a cassette 230a according to an embodiment, comprising a plurality of reference indicators 131a, 131b, 131c.

A carrier tape 250a comprising a plurality of reference indicators 131a, 131b, 131c is arranged on a first spool **301a** and a second spool **302a.** In the illustrated embodiment, the carrier tape 250a is covered with a sealing tape **310a** configured to seal the plurality of reference indicators 131a, 131b, 131c on the carrier tape 250a.

Also, in the illustrated embodiment, a de-sealer **320a** is comprised, for removing the sealing tape 310a from the reference indicators 131a, 131b, 131c on the carrier tape 250a when the relevant reference indicator 131a, 131b, 131c is adjusted into a position aligned with an optional needle **350a** in the cassette 230a.

The cassette 230a, as already discussed, may comprise a liquid insertion connection **340a** configured to receive the milk sample of the animal 100. The liquid insertion connection 340a thus constitute a connection interface between the cassette 230a and the service module 210, through which the milk sample may be received via a tube element of the service module 210.

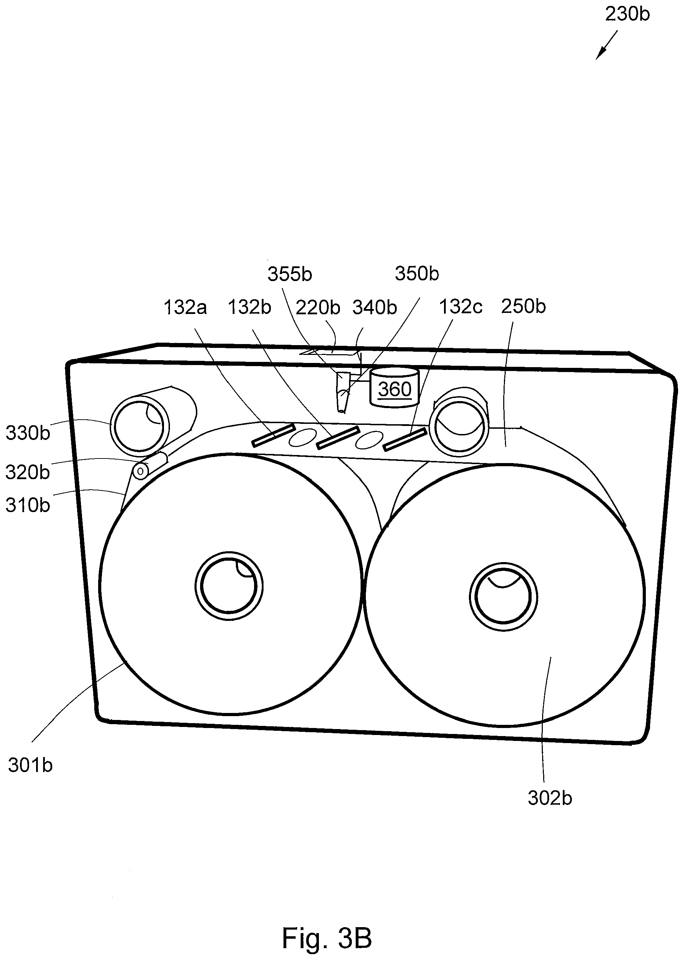

**Figure 3B** illustrates a cassette 230b according to an embodiment, comprising a plurality of measurement indicators 132a, 132b, 132c.

A carrier tape 250b comprising a plurality of measurement indicators 132a, 132b, 132c is arranged on a first spool **301b** and a second spool **302b.** In the illustrated embodiment, the carrier tape 250b is covered with a sealing tape **310b** configured to seal the plurality of measurement indicators 132a, 132b, 132c on the carrier tape 250b.

Also, in the illustrated embodiment, a de-sealer **320b** is comprised, for removing the sealing tape 310b from the measurement indicators 132a, 132b, 132c on the carrier tape 250b when the relevant measurement indicator 132a, 132b, 132c is adjusted into a position aligned with an optional needle **350b** in the cassette 230b.

The cassette 230b, as already discussed, may comprise a liquid insertion connection **340b** configured to receive the milk sample of the animal 100. The liquid insertion connection 340b thus constitute a connection interface between the cassette 230b and the service module 210, through which the milk sample may be received via a tube element of the service module 210.

An advantage with having separate cassettes, one first cassette 130a for the reference indicators 131a, 131b, 131c and a second cassette 130b for the measurement indicators 132a, 132b, 132c is that milk samples may be applied simultaneously on the reference indicator 131 and the measurement indicator 132, which reduces the total measurement time, in comparison with performing sequential application of milk samples on the reference indicator 131 and the measurement indicator 132 with a tape adjustment in between.

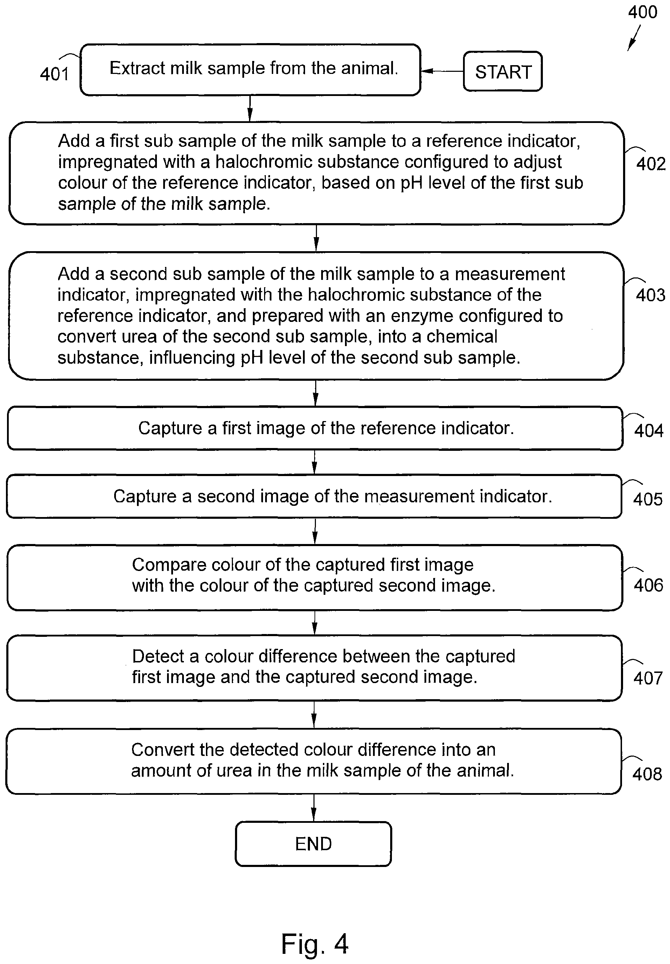

**Figure 4** illustrates a method **400,** aiming at measuring urea in a milk sample of an animal 100. The urea measurement may be made by an arrangement connected to a milking equipment 110 of the animal 100, e.g. on a farm.

In order to be able to measure the urea in the milk sample of the animal 100, the method 400 may comprise a number of steps **401-408.** However, some of these steps 401-408 may be performed solely in some alternative embodiments. Further, the described method steps 401-408 may be performed in a somewhat different chronological order than the numbering suggests. The method 400 may comprise the subsequent steps:
**Step 401** comprises extracting the milk sample from the animal 100. The milk sample is a representative milk sample.

The milk sample may be extracted directly from a milking equipment 110 for milking the animal 100.

**Step 402** comprises adding a first sub sample of the extracted 401 milk sample to a reference indicator 131, impregnated with a halochromic substance configured to adjust colour of the reference indicator 131, based on level of hydroxide ions of the first sub sample of the milk sample.

**Step 403** comprises adding a second sub sample of the milk sample to a measurement indicator 132, impregnated with the halochromic substance of the reference indicator 131, and prepared with an enzyme configured to convert urea of the second sub sample, into at least one chemical substance, which in turn will influence the level of hydroxide ions of the second sub sample.

The enzyme prepared on the measurement indicator 132 may in some embodiments comprise a hydrolytic enzyme, such as e.g. urease.

The at least one chemical substance may comprise ammonia and wherein the level of hydroxide ions of the second sub sample may be increased by the ammonia.

The enzyme may have a buffer capacity lower than a threshold level in some embodiments.

**Step 404** comprises capturing a first image of the reference indicator 131. The first image may be captured after an incubation time period in some embodiments, after addition of the first sub sample of the milk sample to the reference indicator 131. The incubation time period may be an interval in some embodiments, such as e.g. 2-7 minutes, 4-6 minutes, 5 minutes, etc.

**Step 405** comprises capturing a second image of the measurement indicator 132. The second image may be captured after an incubation time period in some embodiments, after addition of the second sub samples of the milk sample to the measurement indicator 132. The incubation time period may be an interval in some embodiments, such as e.g. 2-7 minutes, 4-6 minutes, 5 minutes, etc.

**Step 406** comprises comparing the colour of the captured 404 first image with the colour of the captured 405 second image.

**Step 407** comprises detecting a colour difference between the captured 404 first image and the captured 405 second image.

**Step 408** comprises converting the detected 407 colour difference into an amount of urea in the milk sample of the animal 100.

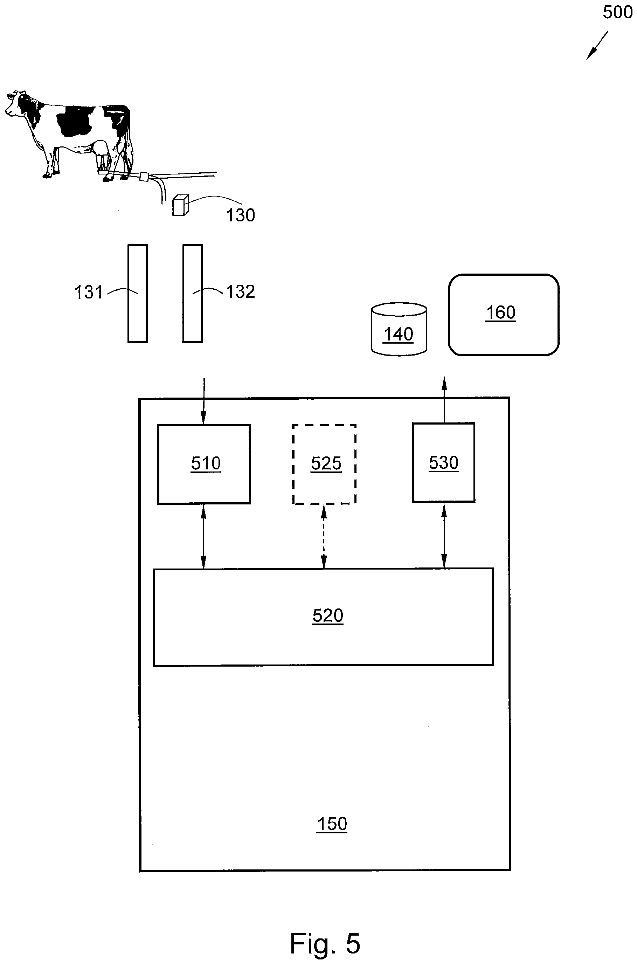

**Figure 5** illustrates an arrangement **500** for measuring urea in a milk sample of an animal 100.

The arrangement 500 comprises a reference indicator 131, impregnated with a halochromic substance configured to adjust colour of the reference indicator 131, based on level of hydroxide ions of a first sub sample of the milk sample, when added to the reference indicator 131. Further the arrangement 500 also comprises a measurement indicator 132, impregnated with the halochromic substance of the reference indicator 131, and prepared with an enzyme configured to convert urea of a second sub sample of the milk sample, when added to the measurement indicator 132, into at least one chemical substance, which in turn will influence the level of hydroxide ions of the second sub sample. Additionally, the arrangement 500 also comprises a sensor 130 configured to capture a first image of the reference indicator 131, and a second image of the measurement indicator 132. Furthermore, the arrangement 500 comprises a control unit 150 configured to determine a colour difference between the first image of the reference indicator 131 and the second image of the measurement indicator 132. The control unit 150 is also configured to convert the determined colour difference into an amount of urea in the milk sample of the animal 100.

In some embodiments, the enzyme prepared on the measurement indicator 132 may comprise a hydrolytic enzyme. The enzyme prepared on the measurement indicator 132 may in some embodiments comprise urease. The chemical substance that the enzyme is converting urea if the second sub sample of the milk sample into, may comprise ammonia in some embodiments. Further, the level of hydroxide ions of the second sub sample may be increased by the ammonia. The enzyme may have a buffer capacity lower than a threshold level in some embodiments.

In some embodiments of the arrangement 500, the reference indicator 131 and the measurement indicator 132 may be maintained within an isolated climate environment 230. Thereby a more predictable and reliable result may be achieved, as the risk of contamination of the reference indicator 131 and/ or the measurement indicator 132 is reduced.

The reference indicator 131 may be maintained in a first cassette 230a and the measurement indicator 132 may be maintained in a second cassette 230b, in some embodiments. However, in some alternative embodiments, the reference indicator 131 and the measurement indicator 132 may be maintained in a common cassette 230.

Further, the arrangement 500 may according to some embodiments be configured for extracting the milk sample is extracted from a milking equipment 110 for milking the animal 100 and wherein the arrangement 500 may be connected to the milking equipment 110.

The control unit 150 may comprise a receiver **510** configured to receive information from the sensor 130, i.e. captured images of the reference indicator 131 and/ or the measurement indicator 132.

The control unit 150 also comprises a processing circuitry **520** configured for performing various calculations for conducting a computer program.

Such processing circuitry 520 may comprise one or more instances of a processing circuit, i.e. a Central Processing Unit (CPU), a processing unit, a processing circuit, a processor, an Application Specific Integrated Circuit (ASIC), a microprocessor, or other processing logic that may interpret and execute instructions. The herein utilised expression "processor" may thus represent a processing circuitry comprising a plurality of processing circuits, such as, e.g., any, some or all of the ones enumerated above.

Furthermore, the control unit 150 may comprise a memory **525** in some embodiments. The optional memory 525 may comprise a physical device utilised to store data or programs, i.e., sequences of instructions, on a temporary or permanent basis. According to some embodiments, the memory 525 may comprise integrated circuits comprising silicon-based transistors. The memory 525 may comprise e.g. a memory card, a flash memory, a USB memory, a hard disc, or another similar volatile or non-volatile storage unit for storing data such as e.g. ROM (Read-Only Memory), PROM (Programmable Read-Only Memory), EPROM (Erasable PROM), EEPROM (Electrically Erasable PROM), etc. in different embodiments.

Further, the control unit 150 may comprise a signal transmitter **530.** The signal transmitter 530 may be configured for transmitting signals via a wired or wireless communication interface to the communication unit 160 of the operator, possibly via a transceiver; and/ or to the database 140.

Furthermore, a computer program comprising instructions to perform measurements of urea in a milk sample of an animal 100 may be performed in the control unit 150.

The computer program mentioned above may be provided for instance in the form of a computer-readable medium, i.e. a data carrier carrying computer program code for performing at least some of the computer program steps, according to some embodiments when being loaded into the one or more processing circuitries 520 of the control unit 150. The data carrier may be, e.g., a hard disk, a CD ROM disc, a memory stick, an optical storage device, a magnetic storage device or any other appropriate medium such as a disk or tape that may hold machine readable data in a non-transitory manner. The computer program may furthermore be provided as computer program code on a server and downloaded to the control unit 150 remotely, e.g. over an Internet or an intranet connection.

The embodiments, or parts thereof, illustrated in Figure 1, Figure 2, Figure 3A, Figure 3B, Figure 4 and/ or Figure 5 may with advantage be combined with each other for achieving further benefits.

The terminology used in the description of the embodiments as illustrated in the accompanying drawings is not intended to be limiting of the described arrangement 500, method 400, cassette 230, service module 210, control unit 150, measurement indicator 132 and/ or computer program. Various changes, substitutions and/ or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g. a processor may fulfil the functions of several items recited in the claims. The mere fact that certain measures or features are recited in mutually different dependent claims, illustrated in different figures or discussed in conjunction with different embodiments does not indicate that a combination of these measures or features cannot be used to advantage. A computer program may be stored/ distributed on a suitable medium, such as an optical storage medium or a solid-state medium supplied together with or as part of other hardware, but may also be distributed in other forms such as via Internet or other wired or wireless communication system.

## Claims

1. An arrangement (500) configured to measure urea in a milk sample of an animal (100); which arrangement (500) comprises:
a reference indicator (131), impregnated with a halochromic substance configured to adjust colour of the reference indicator (131), based on level of hydroxide ions of a first sub sample of the milk sample, when added to the reference indicator (131);
a measurement indicator (132), impregnated with the halochromic substance of the reference indicator (131), and prepared with an enzyme configured to convert urea of a second sub sample of the milk sample, when added to the measurement indicator (132), into at least one chemical substance, which in turn will influence the level of hydroxide ions of the second sub sample;
a sensor (130) configured to capture a first image of the reference indicator (131), and a second image of the measurement indicator (132); and
a control unit (150) configured to determine a colour difference between the first image of the reference indicator (131) and the second image of the measurement indicator (132); and configured to convert the determined colour difference into an amount of urea in the milk sample of the animal (100).

2. The arrangement (500) according to claim 1, wherein the enzyme prepared on the measurement indicator (132) comprises a hydrolytic enzyme.

3. The arrangement (500) according to any one of the preceding claims, wherein:
the enzyme prepared on the measurement indicator (132) is urease;
the chemical substance comprises ammonia; and
the level of hydroxide ions of the second sub sample is increased by the ammonia.

4. The arrangement (500) according to any one of the preceding claims, wherein the reference indicator (131) and the measurement indicator (132) are maintained within an isolated climate environment (230).

5. The arrangement (500) according to any one of the preceding claims; wherein the reference indicator (131) is maintained in a first cassette (230a) and the measurement indicator (132) is maintained in a second cassette (230b).

6. The arrangement (500) according to any one of claims 1-4; wherein the reference indicator (131) and the measurement indicator (132) are maintained in a common cassette (230).

7. The arrangement (500) according to any one of the preceding claims; wherein the milk sample is extracted from a milking equipment (110) for milking the animal (100) and wherein the arrangement (500) is connected to the milking equipment (110).

8. A method (400) to measure urea in a milk sample of an animal (100), which method (400) comprises the steps of:
extracting (401) the milk sample from the animal (100);
adding (402) a first sub sample of the extracted (401) milk sample to a reference indicator (131), impregnated with a halochromic substance configured to adjust colour of the reference indicator (131), based on level of hydroxide ions of the first sub sample of the milk sample;
adding (403) a second sub sample of the milk sample to a measurement indicator (132), impregnated with the halochromic substance of the reference indicator (131), and prepared with an enzyme configured to convert urea of the second sub sample, into at least one chemical substance, which in turn will influence the level of hydroxide ions of the second sub sample;
capturing (404) a first image of the reference indicator (131);
capturing (405) a second image of the measurement indicator (132);
comparing (406) the colour of the captured (404) first image with the colour of the captured (405) second image;
detecting (407) a colour difference between the captured (404) first image and the captured (405) second image;
converting (408) the detected (407) colour difference into an amount of urea in the milk sample of the animal (100).

9. The method (400) according to claim 8, wherein the first image and the second image, respectively, are captured (404, 405) after an incubation time period, after addition of the respective first and second sub samples of the milk sample to the reference indicator (131) and the measurement indicator (132), respectively.

10. The method (400) according to any one of claim 8 or claim 9, wherein the enzyme prepared on the measurement indicator (132) comprises a hydrolytic enzyme.

11. The method (400) according to any one of claims 8-10, wherein:
the enzyme prepared on the measurement indicator (132) is urease;
the chemical substance comprises ammonia; and
the level of hydroxide ions of the second sub sample is increased by the ammonia.

12. The method (400) according to any one of claims 8-11, wherein the milk sample is extracted (401) from a milking equipment (110) for milking the animal (100).

## Patentansprüche

1. Anordnung (500), die konfiguriert ist, um Harnstoff in einer Milchprobe eines Tieres (100) zu messen; wobei die Anordnung (500) Folgendes umfasst:
einen Referenzindikator (131), der mit einer halochromen Substanz getränkt ist, die konfiguriert ist, um eine Farbe des Referenzindikators (131) basierend auf einem Gehalt von Hydroxidionen einer ersten Teilprobe der Milchprobe anzupassen, wenn sie zu dem Referenzindikator (131) hinzugefügt wird;
einen Messindikator (132), der mit der halochromen Substanz des Referenzindikators (131) getränkt und mit einem Enzym hergestellt ist, das konfiguriert ist, um Harnstoff einer zweiten Teilprobe der Milchprobe, wenn sie zu dem Messindikator (132) hinzugefügt wird, in wenigstens eine chemische Substanz umzusetzen, die wiederum den Gehalt von Hydroxidionen der zweiten Teilprobe beeinflusst;
einen Sensor (130), der konfiguriert ist, um ein erstes Bild des Referenzindikators (131) und ein zweites Bild des Messindikators (132) aufzunehmen; und
eine Steuereinheit (150), die konfiguriert ist, um einen Farbunterschied zwischen dem ersten Bild des Referenzindikators (131) und dem zweiten Bild des Messindikators (132) zu bestimmen; und konfiguriert ist, um den bestimmten Farbunterschied in eine Harnstoffmenge in der Milchprobe des Tieres (100) umzusetzen.

2. Anordnung (500) nach Anspruch 1, wobei das Enzym, das auf dem Messindikator (132) hergestellt wird, ein hydrolytisches Enzym umfasst.

3. Anordnung (500) nach einem der vorhergehenden Ansprüche, wobei:
das Enzym, das auf dem Messindikator (132) hergestellt wird, Urease ist;
die chemische Substanz Ammoniak umfasst; und
der Gehalt von Hydroxidionen der zweiten Teilprobe durch das Ammoniak erhöht wird.

4. Anordnung (500) nach einem der vorhergehenden Ansprüche, wobei der Referenzindikator (131) und der Messindikator (132) innerhalb einer isolierten Klimaumgebung (230) gehalten werden.

5. Anordnung (500) nach einem der vorhergehenden Ansprüche; wobei der Referenzindikator (131) in einer ersten Kassette (230a) gehalten wird und der Messindikator (132) in einer zweiten Kassette (230b) gehalten wird.

6. Anordnung (500) nach einem der Ansprüche 1-4; wobei der Referenzindikator (131) und der Messindikator (132) in einer gemeinsamen Kassette (230) gehalten werden.

7. Anordnung (500) nach einem der vorhergehenden Ansprüche; wobei die Milchprobe aus einer Melkausrüstung (110) zum Melken des Tieres (100) extrahiert wird und wobei die Anordnung (500) mit der Melkausrüstung (110) verbunden ist.

8. Verfahren (400) zum Messen von Harnstoff in einer Milchprobe eines Tieres (100), wobei das Verfahren (400) die folgenden Schritte umfasst:
Extrahieren (401) der Milchprobe aus dem Tier (100);
Hinzufügen (402) einer ersten Teilprobe der extrahierten (401) Milchprobe zu einem Referenzindikator (131), der mit einer halochromen Substanz getränkt ist, die konfiguriert ist, um die Farbe des Referenzindikators (131) basierend auf dem Gehalt von Hydroxidionen der ersten Teilprobe der Milchprobe anzupassen;
Hinzufügen (403) einer zweiten Teilprobe der Milchprobe zu einem Messindikator (132), der mit der halochromen Substanz des Referenzindikators (131) getränkt und mit einem Enzym hergestellt ist, das konfiguriert ist, um Harnstoff der zweiten Teilprobe in wenigstens eine chemische Substanz umzusetzen, die wiederum den Gehalt von Hydroxidionen der zweiten Teilprobe beeinflusst;
Aufnehmen (404) eines ersten Bildes des Referenzindikators (131);
Aufnehmen (405) eines zweiten Bildes des Messindikators (132);
Vergleichen (406) der Farbe des aufgenommenen (404) ersten Bildes mit der Farbe des aufgenommenen (405) zweiten Bildes;
Erfassen (407) eines Farbunterschiedes zwischen dem aufgenommenen (404) ersten Bild und dem aufgenommenen (405) zweiten Bild;
Umsetzen (408) des erfassten (407) Farbunterschieds in eine Harnstoffmenge in der Milchprobe des Tieres (100).

9. Verfahren (400) nach Anspruch 8, wobei das erste Bild beziehungsweise das zweite Bild nach einer Inkubationszeitdauer nach Zugabe der jeweiligen ersten und der zweiten Teilprobe der Milchprobe zu dem Referenzindikator (131) beziehungsweise dem Messindikator (132) aufgenommen (404, 405) werden.

10. Verfahren (400) nach einem der Ansprüche 8 oder 9, wobei das Enzym, das auf dem Messindikator (132) hergestellt wird, ein hydrolytisches Enzym umfasst.

11. Verfahren (400) nach einem der Ansprüche 8-10, wobei:
das Enzym, das auf dem Messindikator (132) hergestellt wird, Urease ist;
die chemische Substanz Ammoniak umfasst; und
der Gehalt von Hydroxidionen der zweiten Teilprobe durch das Ammoniak erhöht wird.

12. Verfahren (400) nach einem der Ansprüche 8-11, wobei die Milchprobe aus einer Melkausrüstung (110) zum Melken des Tieres (100) extrahiert (401) wird.

## Revendications

1. Agencement (500) configuré pour mesurer l'urée dans un échantillon de lait d'un animal (100) ; lequel agencement (500) comprend :
un indicateur de référence (131), imprégné d'une substance halochromique conçue pour régler la couleur de l'indicateur de référence (131), sur la base du niveau d'ions hydroxyde d'un premier sous-échantillon de l'échantillon de lait, lorsqu'il est ajouté à l'indicateur de référence (131) ;
un indicateur de mesure (132), imprégné de la substance halochromique de l'indicateur de référence (131), et préparé avec une enzyme conçue pour convertir l'urée d'un second sous-échantillon de l'échantillon de lait, lorsqu'elle est ajoutée à l'indicateur de mesure (132), en au moins une substance chimique, qui à son tour influencera le niveau d'ions d'hydroxyde du second sous-échantillon ;
un capteur (130) configuré pour capturer une première image de l'indicateur de référence (131), et une seconde image de l'indicateur de mesure (132) ; et
une unité de commande (150) configurée pour déterminer une différence de couleur entre la première image de l'indicateur de référence (131) et la seconde image de l'indicateur de mesure (132) ; et configuré pour convertir la différence de couleur déterminée en une quantité d'urée dans l'échantillon de lait de l'animal (100).

2. Agencement (500) selon la revendication 1, l'enzyme préparée sur l'indicateur de mesure (132) comprenant une enzyme hydrolytique.

3. Agencement (500) selon l'une quelconque des revendications précédentes :
l'enzyme préparée sur l'indicateur de mesure (132) étant l'uréase ;
la substance chimique comprenant de l'ammoniac ; et
le niveau d'ions hydroxyde du second sous-échantillon étant augmenté par l'ammoniac.

4. Agencement (500) selon l'une quelconque des revendications précédentes, l'indicateur de référence (131) et l'indicateur de mesure (132) étant maintenus à l'intérieur d'un environnement climatique isolé (230).

5. Agencement (500) selon l'une quelconque des revendications précédentes ; l'indicateur de référence (131) étant maintenu dans une première cassette (230a) et l'indicateur de mesure (132) étant maintenu dans une seconde cassette (230b).

6. Agencement (500) selon l'une quelconque des revendications 1 à 4 ; l'indicateur de référence (131) et l'indicateur de mesure (132) étant maintenus dans une cassette commune (230).

7. Agencement (500) selon l'une quelconque des revendications précédentes ;
l'échantillon de lait étant extrait d'un équipement de traite (110) destiné à traire l'animal (100) et l'agencement (500) étant relié à l'équipement de traite (110).

8. Procédé (400) pour mesurer l'urée dans un échantillon de lait d'un animal (100), lequel procédé (400) comprend les étapes consistant à :
extraire (401) l'échantillon de lait de l'animal (100) ;
ajouter (402) un premier sous-échantillon de l'échantillon de lait extrait (401) à un indicateur de référence (131), imprégné d'une substance halochromique conçue pour régler la couleur de l'indicateur de référence (131), sur la base du niveau d'ions hydroxyde du premier sous-échantillon de l'échantillon de lait ;
ajouter (403) un second sous-échantillon de l'échantillon de lait à un indicateur de mesure (132), imprégné de la substance halochromique de l'indicateur de référence (131), et préparé avec une enzyme configurée pour convertir l'urée du second sous-échantillon, en au moins une substance chimique, qui à son tour influencera le niveau d'ions d'hydroxyde du second sous-échantillon ;
capturer (404) une première image de l'indicateur de référence (131) ;
capturer (405) une seconde image de l'indicateur de mesure (132) ;
comparer (406) la couleur de la première image capturée (404) avec la couleur de la seconde image capturée (405) ;
détecter (407) une différence de couleur entre la première image capturée (404) et la seconde image capturée (405) ;
convertir (408) la différence de couleur détectée (407) en une quantité d'urée dans l'échantillon de lait de l'animal (100).

9. Procédé (400) selon la revendication 8, la première image et la seconde image, respectivement, étant capturées (404, 405) après une période d'incubation, après ajout des premier et second sous-échantillons respectifs de l'échantillon de lait à l'indicateur de référence (131) et à l'indicateur de mesure (132), respectivement.

10. Procédé (400) selon l'une quelconque des revendications 8 ou 9, l'enzyme préparée sur l'indicateur de mesure (132) comprenant une enzyme hydrolytique.

11. Procédé (400) selon l'une quelconque des revendications 8 à 10 :
l'enzyme préparée sur l'indicateur de mesure (132) étant l'uréase ;
la substance chimique comprenant de l'ammoniac ; et
le niveau d'ions hydroxyde du second sous-échantillon étant augmenté par l'ammoniac.

12. Procédé (400) selon l'une quelconque des revendications 8 à 11, l'échantillon de lait étant extrait (401) d'un équipement de traite (110) destiné à traire l'animal (100).
